Europäisches Patentamt

European Patent Office

Office européen des brevets

(19)

(11) Publication number: **0 332 826**

**A1**

(12)

# EUROPEAN PATENT APPLICATION

(21) Application number: **89101629.7**

(22) Date of filing: **31.01.89**

(51) Int. Cl.4: **A61K 9/08 , A61K 47/00**

The title of the invention has been amended (Guidelines for Examination in the EPO, A-III, 7.3).

(30) Priority: **03.02.88 IL 85312**

(43) Date of publication of application:
**20.09.89 Bulletin 89/38**

(84) Designated Contracting States:
**AT BE CH DE ES FR GB GR IT LI LU NL SE**

(71) Applicant: **Teva Pharmaceutical Industries Limited**
**Har Hotzvim behind Sanhedria Ha'Murhevet**
**Jerusalem(IL)**

(72) Inventor: **Tashma, Zev**
**2 Shahal Street**
**Jerusalem 93701(IL)**

(74) Representative: **Brown, John David et al**
**FORRESTER & BOEHMERT**
**Widenmayerstrasse 4/I**
**D-8000 München 22(DE)**

(54) Injectable pharmaceutical compositions having improved stability, and methods of producing the same.

(57) Improved pharmaceutical compositions having a long term shelf life, which comprises certain pharmacologically active water-hydrolyzable derivatives of $\alpha$-amino- and $\alpha$- 5 hydroxy-carboxylic acids and a solvent medium containing an aqueous component, characterized in that at least a major proportion by volume of the ordinary water normally used in formulating such compositions is replaced by deuterium oxide.

EP 0 332 826 A1

## BACKGROUND OF THE INVENTION

Injectable pharmaceutical compositions usually require the presence of water. In certain cases, however, water has a destabilizing influence on the pharmaceutically active ingredients of such compositions, e.g. it may cause such ingredients to hydrolyze and in consequence lose their pharmacological potency. In these cases, where the compositions are to be used in clinics or hospitals, one solution of the problem is to store the active ingredients in a form in which injectable compositions can be readily formulated therefrom by addition of water, and to prepare such compositions only on demand. There exist, however, situations in which such a solution to the problem of destabilization in presence of water cannot be applied. For example, for reasons of public health, in order to combat or prevent a disease, it may be desirable to maintain a stock of the order of hundred of thousands, or even millions of units, of injectable pharmaceutical compositions. Similarly, in order to provide an antidote in case of chemical warfare it may be desirable to be able to distribute very large number of injectable compositions which are ready for use to the civilian population or to military personnel.

Moreover, for reasons of convenience or economy, it may be desirable to store these injectable pharmaceutical compositions for long terms. The expression "long term", when used herein in this context, means a period of at least one year. That is to say, it is contemplated that such injectable compositions may be stored under ambient conditions with a tolerable degree of deterioration of their pharmacological effectiveness, for periods of at least one year, possibly even for several years.

It has previously been proposed to replace some of the water in injectable compositions by propylene glycol. Thus, in U.S. 4,212,886 (Homan), the contents of which are to be regarded as incorporated herein by reference, there is described and claimed a pharmaceutical composition comprising (as active ingredient) benactyzine hydrochloride dissolved in a solvent mixture of 30-50% propylene glycol and 70-50% water, by volume, subject to the qualifications that the amount of propylene glycol is sufficient to increase the stability of the active ingredient over that in water along and that the amount of water is sufficient to prevent dehydration of the active ingredient. Additionally, ethanol may be present in order to reduce the viscosity so as to render the composition more easily injectable; thus, the solvent mixture may comprise by volume, 50% propylene glycol, 40% water and 10% ethanol.

However, Homan's compositions suffer from a number of disadvantages. Firstly, the degree of stabilization appears to be insufficient for practical application. Secondly, over the long term, propylene glycol may give rise to undesirable degradation products per se and also to undesirable reaction products with the active ingredients, thereby detracting from the pharmaceutical purity of the compositions. Thirdly, the viscosity characteristics of aqueous solutions of propylene glycol are inconvenient, both from a handling and an administration point of view.

Siegel et al J. Pharm. Sci. 53: 978 (1964) and 51: 1166 (1962) proposed to stabilize solutions of procaine, which may be suitable for ophtalmic use, by substituting the ordinary water in these solutions by deuterium oxide. Siegel's experiments relate to the stabilization of these solutions in the alkaline pH range at 40° C; in no case does such stabilization lead to a half-life greater than 115 hours. Thus, there is no suggestion that it could be practicable to use deuterium oxide for long term stabilization of pharmaceutical compositions of any kind.

In accordance with the invention it has now been surprisingly discovered that the stability of certain aqueous pharmaceutical compositions intended for long term storage can be enhanced by replacing at least a major part of the ordinary water in such compositions by deuterium oxide.

## SUMMARY OF THE INVENTION

It is, therefore an object of the present invention to stabilize and thus prolong the long term shelf-life of certain injectable pharmaceutical compositions by replacing the greater part of the ordinary water normally used therein by deuterium oxide.

Another object of the invention is to significantly reduce the amount of ordinary water present in certain injectable pharmaceutical compositions, by replacing at least a major part thereof by deuterium oxide, whereby the deteriorative reaction with water of the active ingredient(s) in the long term will be significantly retarded.

Other objects of the invention will become apparent from the following description.

Throughout the present specification and claims, "%" in relation to ingredients of solvent media is intended to signify % by volume.

The present invention accordingly provides, in one aspect, an injectable pharmaceutical composition having a long term shelf life, which composition comprises:

at least one pharmacologically active ingredient which is susceptible to deterioration due to the presence of water, selected from the group consisting of water-hydrolyzable derivatives of α-amino- and α-hydroxy- carboxylic acids, pharmaceutically acceptable acid addition salts and alkali metal, alkaline earth metal and ammonium salts of such derivatives; and

a solvent medium containing an aqueous component, said aqueous component comprising at least a major proportion by volume of deuterium oxide.

In another aspect, the invention provides a method of producing an injectable pharmaceutical composition having a long term shelf-life, which comprises dissolving at least one pharmacologically active ingredient which is susceptible to deterioration due to the presence of water, selected from the group consisting of water-hydrolyzable derivatives of α-amino and α-hydroxy- carboxylic acids, pharmaceutically acceptable acid addition salts and alkali metal, alkaline earth metal and ammonium salts of such derivatives; in a solvent medium containing an aqueous component, said aqueous component comprising at least a major proportion by volume of deuterium oxide.

The term "ordinary water", as used herein, is intended to mean water suitable for preparing injectable pharmaceutical formulations, but which in any event contains no more than the normally occurring amount of deuterium oxide. As previously mentioned, "long term" (storage or shelf life) in the present context signifies a period of at least one year. Thus, in accordance with the present invention, the injectable pharmaceutical compositions will have a storage or shelf-life of at least one year, and the initial storage or shelf-life, whatever it may be, is prolonged by the replacement of at least the major part by volume of the ordinary water present, by deuterium oxide.

## DETAILED DESCRIPTION OF THE INVENTION

Contrary to the prior art teaching, it has been found in accordance with the present invention that there is no minimum amount of ordinary water required "sufficient to prevent dehydration of the active ingredient". In accordance with the invention, 99.9 or 100% of the ordinary water present in the injectable composition could be replaced by deuterium oxide, but for practical reasons there will usually be present a small residual amount of ordinary water. Thus from a practical and economic point of view, the aqueous component of the solvent medium in the injectable pharmaceutical compositions may contain, say, about 98% or 99% deuterium oxide, the balance being ordinary water.

However, since in accordance with the invention a major proportion of the ordinary water present in the aqueous component of the solvent medium is replaced by deuterium oxide, it will be evident that other percentages than those just mentioned are viable. Thus, for example, the aqueous component of the injectable composition according to the invention may comprise from about 75 to about 100%, preferably from about 90 to about 100% deuterium oxide and from about 25 to about 0%, preferably from about 10 to about 0% ordinary water. Most preferably, the aqueous component comprises from about 95 to about 100% deuterium oxide and from about 5 to about 0% ordinary water. It is especially preferred that in the compositions according to the invention, the aqueous component comprises from about 98 to about 100% deuterium oxide and from about 2 to about 0% ordinary water, and more particularly from about 99 to about 99.75% deuterium oxide and from about 1 to about 0.25% ordinary water.

As will be appreciated by those skilled in the pharmaceutical art, the solvent medium may be wholly comprised of an aqueous component or, alternatively, the solvent medium may be a mixture of an aqueous component with a water-miscible non-aqueous liquid.

As is well known, deuterium oxide is itself both stable and effectively non-toxic (in the amounts presently contemplated for use herein, e.g. up to about 10 ml). It is therefore an ideal solvent medium for the present purposes.

Deuterium oxide is present in small amounts in ordinary water, including the water content of the human body; an average adult male contains about 6 ml deuterium oxide. It is close in chemical and physical properties to ordinary water. In connection with its physiological properties, it is to be noted that deuterium oxide has been routinely used over a period of years for measuring total body water content. For this purpose, quantities up to about 100 ml are used. In a published experiment (Taylor et al, Arch. Path., 81: 213 (1966)), considerable amounts of deuterium oxide were administered continuously to humans over a period of about 6 to 7 weeks, with little or no notable ill effects.

The deuterium oxide according to the invention should preferably pass the tests for ordinary water for injection as required by a recent edition of a recognized pharmacopoeia, adapted, if and as necessary, for the specific characteristics of deuterium oxide.

The use of deuterium oxide in accordance with the present invention is believed to have the following advantages over the possible use of other solvents (except ordinary water) for the same purpose:

1. it is far less toxic than other known solvents, except for ordinary water;

2. no new decomposition products due to drug-solvent interactions are to be expected, besides those which are known from ordinary water (or analogues of such known products);

3. no contamination due to dehydration, resinification, oxidation or other chemical changes of the solvent per se, is to be expected, as compared with the possible use of non-aqueous solvents; and

4. unlike most other non-aqueous solvents, deuterium oxide can be brought to a high and uniform standard of chemical and microbiologtical purity, by adapting the pharmacopoeia standards and tests for ordinary water for injection.

It is preferred that the compositions of the present invention have a pH within the range of about 1.5 to about 5.5. The manner of adjustment of pH values is of course well known to those skilled in the art.

The compositions of the present invention may contain additionally at least one further pharmacologically active ingredient, besides the at least one carboxylic acid derivative susceptible to hydrolysis as defined herein.

As previously noted, the at least one active ingredient is a water-hydrolyzable derivative of an α-amino- or α-hydroxy-carboxylic acid (including salts thereof). Such derivatives include, particularly, esters and amides, including N-substituted amides. The carboxylic acid derivative is also characterized by the fact that it contains an α-amino or α-hydroxy group. A presently preferred sub-group comprises the α-hydroxy- carboxylic acid derivatives. An exemplary member of this sub-group is benactyzine and its pharmaceutically acceptable acid addition salts, e.g. the HC1 salt. In the case of benactyzine and its pharmaceutically acceptable acid addition salts, the pH of the composition of the invention is preferably within the range of about 2.0 to about 4.0, more preferably within the range of about 2.7. to about 2.8.

As already indicated generally, such compositions which contain benactyzine (or its salts) may contain additionally at least one further pharmacologically active ingredient; the latter may, for example, be selected from cholinolytic drugs, pharmaceutically accepted oximes and pharmaceutically acceptable salts thereof. It is preferably selected from atropine, pharmaceutically acceptable salts thereof and trimedoxime (which is also known as "TMB-4"). Atropine may be used as the sulfate salt, for example. Trimedoxime is a diquaternary salt in which the anion may be, e.g., bromide.

The medical treatment of an acute exposure to certain organophosphorus insecticides or nerve gases, which are anticholinesterase agents, demands immediate administration of cholinolytics, to control the excessive cholinergic stimulation. For this purpose, it is well known to use atropine, and a combination of atropine with benactyzine. Addition of oximes to cholinolytic drugs has the effect of reactivating and restoring the cholinesterase activity. Zvirblis and Ellin, in J. Pharm. Sci. 71:321 (1982), described a 3-component mixture containing 2 ml of injection solution: 40 mg of trimedoxime bromide, 1 mg of atropine sulfate and 4.1 mg of benactyzine hydrochloride. It will be evident to those skilled in the art that the proportions and quantities of the ingredients in such a three-component mixture can be varied. Other related drug mixtures are described in Schenk et al., Arch. Toxicol., 36:71 (1976). The contents of both the Zvirblis and Ellin article and the Schenk et al. article are to be regarded as incorporated herein by reference.

The susceptibility of benactyzine to hydrolysis (which under optimal conditions of pH 2.7-2.8 may amount to about 20% per year at 25°C and about 2% per year at 5°C according to Zvirblis and Ellin), detracts from its usefulness as a component of a large scale antidote to organophosphorus poisoning, because the evident necessity for refrigeration requires considerable expense and logistics to store the quantity of units required. In accordance with the present invention, making use of deuterium oxide, the storage characteristics of benactyzine, whether kept alone or in combination with other ingredients, are considerably enhanced.

The invention moreover extends to an automatic injector characterized by the presence therein of an injectable composition according to the invention. Normally, the automatic injector will be a single stage injector, i.e. comprising a single compartment containing the injectable composition. However, in cases where an active ingredient which is beneficially stabilized in the long term is to be used together with one or more other active ingredients which are sufficiently stable (without substitution of deuterium oxide for ordinary water), then it will be apparent that economy in the consumption of deuterium oxide may be achieved by use of a multistage automatic injector comprising one compartment containing the relatively less stable active ingredient dissolved in deuterium oxide, and one or more other compartments containing the relatively more stable active ingredients dissolved in ordinary water.

The invention will be illustrated by the following non-limitative examples, in which 99.75% deuterium oxide was used initially. Owing to the hygroscopic nature of this substance, and to the presence of small amounts of ordinary water in the composition ingredients and in the apparatus, the solvent medium in the composition end-products contained about 99% deuterium oxide, the balance being ordinary water. The deuterium oxide should pass the tests for Water for Injection of U.S.P. vol. XXI, adapted as appropriate for deuterium oxide.

## EXAMPLE I

Benactyzine hydrochloride (80 mg) is dissolved in 18 ml of deuterium oxide, the pH is adjusted by known methods (see e.g. A.K. Covington, Analytical Chemistry, 40: 700 (1968)) to 2.7 with strong acid, and the volume is brought to 20 ml with more deuterium oxide. The resulting solution is sterilized by passing through a pharmaceutically acceptable and antimicrobial filtering device, and then divided into 1 ml portions. The resultant composition is suitable for use in a single stage automatic injector, where benactyzine is the only active ingredient to be administered, or in a multistage automatic injector together with relatively more stable active ingredients (such as atropine and trimedoxime) dissolved in ordinary water in one or more further separate compartment(s).

## EXAMPLE II

Benactyzine hydrochloride (40 mg), atropine sulfate (10 mg) and trimedoxime bromide (400 mg) are dissolved in 18 ml of deuterium oxide. The pH is adjusted by known methods to 2.7 with strong acid, and the volume is brought to 20 ml with more deuterium oxide. The resulting solution is sterilized by passing through a pharmaceutically acceptable and antimicrobial filtering device, and then divided into 2 ml portions. The resultant composition is suitable for use in a single stage automatic injector.

It was found that the shelf life at room temperature of the compositions of Examples I and II was considerably enhanced as compared to similar compositions prepared with ordinary water only.

Since it will be apparent to those skilled in the art that many modifications and variations could be made in the particular description of the invention hereinabove, the invention is not to be construed as limited thereby, but rather the invention will be defined only by the claims which follow.

The features disclosed in the foregoing description, in the claims and/or in the accompanying drawings may, both, separately and in any combination thereof, be material for realising the invention in diverse forms thereof.

## Claims

1. An injectable pharmaceutical composition having a long term shelf life, which composition comprises:
at least one pharmacologically active ingredient which is susceptible to deterioration due to the presence of water, selected from the group consisting of water-hydrolyzable derivatives of α-amino- and α-hydroxy- carboxylic acids, pharmaceutically acceptable acid addition salts and alkali metal, alkaline earth metal and ammonium salts of such derivatives; and
a solvent medium containing an aqueous component, said aqueous component comprising at least a major proportion by volume of deuterium oxide.

2. A composition according to claim 1, wherein said aqueous component comprises from about 75 to about 100% by volume of deuterium oxide and from about 25 to about 0% by volume of ordinary water.

3. A composition according to claim 2, wherein said aqueous component comprises from about 90 to about 100% by volume of deuterium oxide and from about 10 to about 0% by volume of ordinary water.

4. A composition according to claim 3, wherein said aqueous component comprises from about 95 to about 100% by volume of deuterium oxide and from about 5 to about 0% by volume of ordinary water.

5. A composition according to claim 4, wherein said aqueous component comprises from about 98 to about 100% by volume of deuterium oxide and from about 2 to about 0% by volume of ordinary water.

6. A composition according to claim 5, wherein said aqueous component comprises from about 99 to about99.75% by volume of deuterium oxide and from about 1 to about 0.25% by volume of ordinary water.

7. A composition according to claim 5, wherein said aqueous component consists of substantially 100% by volume of deuterium oxide.

8. A composition according to any one of claims 1 to 7, having a pH within the range of about 1.5 to about 5.5.

9. A composition according to any one of claims 1 to 8, wherein said solvent medium comprises a mixture of an aqueous component with a water-miscible solvent.

10. A composition according to any one of claims 1 to 9, wherein said at least one pharmacologically active ingredient is an ester of an α-hydroxy-carboxylic acid.

11. A composition according to claim 10, wherein said at least one active ingredient comprises at least one member selected from the group consisting of benactyzine and its pharmaceutically acceptable acid addition salts, and the pH of the composition is within the range of about 2.0 to about 4.0.

12. A composition according to claim 11, having a pH within the range of about 2.7. to about 2.8.

13. A composition according to any one of claims 1 to 12, which contains additionally at least one further pharmacologically active ingredient.

14. A composition according to claim 13, wherein said at least one further pharmacologically active ingredient is selected from the group consisting of cholinolytic drugs, pharmaceutically acceptable oximes and pharmaceutically acceptable salts thereof.

15. A composition according to claim 14, wherein said at least one further pharmacologically active ingredient is selected from the group consisting of atropine, pharmaceutically acceptable salts thereof and trimedoxime.

16. A single stage automatic injector characterised by the presence therein of a composition according to any of claims 1 to 15.

17. A multistage automatic injector characterised by the presence therein of a composition according to any of claims 1 to 15.

18. A method of producing an injectable pharmaceutical composition having a long term shelf-life, which comprises dissolving at least one pharmacologically active ingredient which is susceptible to deterioration due to the presence of water, selected from the group consisting of water-hydrolyzable derivatives of $\alpha$-amino and $\alpha$-hydroxy-carboxylic acids, pharmaceutically acceptable acid addition salts and alkali metal, alkaline earth metal and ammonium salts of such derivatives; in a solvent medium containing an aqueous component, said aqueous component comprising at least a major proportion by volume of deuterium oxide.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | JOURNAL OF PHARMACEUTICAL SCIENCES, vol. 71, no. 3, March 1982, pages 321-325, American Pharmaceutical Association, Washington, DC, US; P. ZVIRBLIS et al.: "Kinetics and stability of a multicomponent organophosphate antidote formulation in glass and plastic" * Whole document * | 1-18 | A 61 K 9/08 A 61 K 47/00 |
| D,A | JOURNAL OF PHARMACEUTICAL SCIENCES, vol. 53, no. 8, August 1964, pages 978-979, American Pharmaceutical Association, Washington, DC, US; F.P. SIEGEL et al.: "Stability of procaine in deuterium oxide" * Whole document * | 1-18 | |
| D,A | US-A-4 212 886 (G. HOMAN) * Whole document * | 1-18 | |
| A | JOURNAL OF PHARMACY AND PHARMACOLOGY, vol. 8, 1956, pages 907-914, Pharmaceutical Society of Great Britain, London, GB; J.P. JEFFERIES et al.: "The determination of benactyzine" * Page 911, last paragraph - page 912, paragraph 1 * | 1-18 | TECHNICAL FIELDS SEARCHED (Int. Cl.4) A 61 K |
| A | CHEMICAL ABSTRACTS, vol. 58, no. 12, 10th June 1963, column 12385c,d, Columbus, Ohio, US; S.G. KUZNETSOV et al.: "Comparative study of rates of hydrolysis of cholinolytically active aminoalkyl esters and thio esters", & ZH. OBSHCH. KHIM. 32, 2026-9(1962) * Abstract * | 1-18 | |

-/-

The present search report has been drawn up for all claims

| Place of search | Date of completion of the search | Examiner |
|---|---|---|
| THE HAGUE | 14-07-1989 | MUELLNERS W. |

EPO FORM 1503 03.82 (P0401)

European Patent Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | JOURNAL OF PHARMACEUTICAL SCIENCES, vol. 68, no. 7, July 1979, pages 856-859, American Pharmaceutical Association, Washington, DC, US; L.A. HULL et al.: "3-Quinuclidinyl benzilate hydrolysis in dilute aqueous solution" * Page 856, left-hand column; page 858, right-hand column - page 859, left-hand column * | 1-18 | |
| A | CHEMICAL REVIEWS, vol. 55, 1955, pages 713-743, American Chemical Society, Easton, PA, US; K.B. WIBERG: "The deuterium isotope effect" * Page 718, paragraph 4 - page 723; page 724, paragraph 3 * | 1-18 | |
| A | JOURNAL OF THE AMERICAN PHARMACEUTICAL ASSOCIATION, vol. 46, no. 9, September 1957, pages 531-535, American Pharmaceutical Association, Washington, DC, US; A.A. KONDRITZER et al.: "Stability of atropine in aqueous solution" * Whole document * | 13-15 | TECHNICAL FIELDS SEARCHED (Int. Cl.4) |
| A | JOURNAL OF PHARMACEUTICAL SCIENCES, vol. 55, no. 11, November 1966, pages 1263-1267, American Pharmaceutical Association, Washington, DC, US; R.I. ELLIN et al.: "Kinetics of deterioration of trimethylene bis-(4-formylpyridinium bromide) dioxime in dilute aqueous solutions" * Whole document * | 13-15 | |

The present search report has been drawn up for all claims

| Place of search | Date of completion of the search | Examiner |
|---|---|---|
| THE HAGUE | 14-07-1989 | MUELLNERS W. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
................................................................
& : member of the same patent family, corresponding
    document

EPO FORM 1503 03.82 (P0401)